# EUROPEAN PATENT SPECIFICATION

(11) **EP 1 433 769 B1**
(45) Date of publication and mention of the grant of the patent: **11.11.2009**
(21) Application number: 02798818.7
(22) Date of filing: 10.09.2002
(51) Int. Cl.: C07C 1/20, C07C 15/085, C07D 301/19, C07D 303/04, C07C 1/22

(54) **PROCESS FOR PRODUCING CUMENE**
VERFAHREN ZUR HERSTELLUNG VON CUMEN
PROCEDE DE FABRICATION DE CUMENE

(30) Priority: 13.09.2001 JP 2001277701
(43) Date of publication of application: 30.06.2004
(73) Proprietor: Sumitomo Chemical Company, Limited, Tokyo 104-8260 (JP)
(72) Inventor: TSUJI, Junpei, Ichihara-shi, Chiba 299-0125 (JP); OKU, Noriaki, Ichihara-shi, Chiba 290-0038 (JP)
(74) Representative: Henkel, Feiler & Hänzel
(86) International application number: PCT/JP2002/009212
(87) International publication number: WO 2003/024900

(56) References cited:
- WO-A1-01/70711
- WO-A1-01/70714
- JP-A- 2001 270 880
- US-A- 3 337 646
- G. BRACA ET AL: IND. ENG. CHEM. RES., vol. 34, no. 7, 1995, pages 2358-2363, XP002295380

## Description

### Technical Field

The present invention relates to a process for producing cumene. More particularly, the present invention relates to a process for producing cumene, which includes subjecting cumyl alcohol to hydrogenolysis in the presence of a copper-based catalyst, and which has excellent characteristics that deterioration of an activity caused by reduction of a partial pressure of hydrogen and poisoning of the catalyst, can be prevented, whole volume of a reactor can be effectively utilized, and a purged amount of a gas can be suppressed to small when hydrogen is recycled.

### Background Art

It is publicly known to produce cumene by hydrogenolysis of cumyl alcohol in the presence of a copper-based catalyst.

US-A-3 337 646 discloses a process for the conversion of an aryl carbinol to the corresponding hydrocarbon which comprises reacting said carbinol with hydrogen in the vapor phase at temperatures from 150°C to 500 °C in the presence of a hydrogenating metal plus modifier catalyst, said hydrogenating metal being a member of the group consisting of Ni, Zn, Cu, Co, Ag and Sn, said modifier being a member of the group consisting of chromium and iron, said catalyst containing 30 to 99% of the hydrogenating metal based on the latter metal plus modifier, the latter being calculated as its lowest valence state oxide and containing 1-15% basic material.

### Disclosure of the Invention

The present inventors have studied regarding use of hydrogen which is used as a raw material for hydrogenolysis of cumyl alcohol, for example, hydrogen separated from a water gas obtained by reaction of methane and steam, and as a result, found that a small amount of carbon monoxide contained in hydrogen had a poisoning action on the catalyst. As a result of additional studies, they found that it was effective to suppress the amount of carbon monoxide to 5% by volume or less, and attained the present invention.

According to present invention, there is provided a process for producing cumene, which includes subjecting cumyl alcohol to hydrogenolysis in the presence of a copper-based catalyst, and which has excellent characteristics that deterioration of an activity caused by reduction of a partial pressure of hydrogen and poisoning of the catalyst, can be prevented, whole volume of a reactor can be effectively utilized, and an amount purged of a gas can be suppressed to small when hydrogen is recycled.

Namely, the present invention relates to a process for producing cumene, which comprises separating and removing carbon monoxide from a water gas to obtain hydrogen not containing carbon monoxide or containing 5% by volume or less of carbon monoxide and subjecting cumyl alcohol to hydrogenolysis using said hydrogen in the presence of a copper-based catalyst.

### Best Mode for Carrying out the Invention

The copper-based catalyst includes copper, Raney copper, copper-chromium, copper-zinc, copper-chromium-zinc, copper-silica, copper-alumina and compounds containing these.

The hydrogenolysis is usually carried out by contacting cumyl alcohol with hydrogen in the presence of the catalyst. The reaction can be carried out in a liquid phase using a solvent or in a gas phase. The solvent should be substantially inert to the reactants and the product. The solvent may be composed of a substance existing in a cumyl alcohol solution used. When, for example, cumyl alcohol is a mixture with cumene as the product, it is possible to use cumene as a solvent without adding a solvent, in particular. Other useful solvents include alkanes (e.g. octane, decane, dodecane) and aromatic monocyclic compounds(e.g. benzene, ethylbenzene, toluene) and the like. The hydrogenolysis temperature is usually 0 to 500°C and preferably 30 to 400°C. In general, the pressure is advantageously 100 to 10000 kPa.

The hydrogenolysis can be advantageously carried out using the catalyst in the form of a slurry or a fixed-bed. The reaction can be carried out by a batch process, a semi-continuous process, or a continuous process.

In the present invention, it is necessary to suppress a content of carbon monoxide in a hydrogen gas to 5% by volume or less, in addition, it is preferable to use hydrogen in which total contents of carbon monoxide and carbon dioxide are suppressed to 15% by volume or less. Further, the content of carbon monoxide is preferably 3% by volume or less.

As a hydrogen resource, hydrogen obtained by using a water gas as a raw material is used.

The water gas is obtained by a catalytic reforming of a hydrocarbon such as methane or cokes with steam at high temperature, and is a mixed gas substantially composed of hydrogen, methane, carbon monoxide and carbon dioxide.

The activity deterioration of the catalyst in long time operation can be suppressed by satisfying the above-described condition. As a method for reducing carbon monoxide and carbon dioxide contained in hydrogen, for example, separation and removal methods such as absorbing removal by means of water, a solvent such as caustic water or an absorbent, adsorbing removal by means of a zeolite or a carbon molecular sieve, removal by means of reaction such as methanation, separation by means of a pressure swing absorbing method(PSA), and separation utilizing a semi-permeable membrane, are illustrated.

The process of the present invention can be conducted in hydrogenolysis step in production of propylene oxide comprising the following steps:
oxidation step: a step of obtaining cumene hydroperoxide by oxidizing cumene,
epoxidation step: a step of obtaining propylene oxide and cumyl alcohol by reacting cumene hydroperoxide obtained in the oxidation step with propylene in an excess amount in a liquid in the presence of a solid catalyst, and
hydrogenolysis step: a step of obtaining cumene by subjecting cumyl alcohol obtained in the epoxidation step to hydrogenolysis, and recycling the cumene to the oxidation step as a raw material for the oxidation step.

The oxidation step is a step for obtaining cumene hydroperoxide by oxidizing cumene. The oxidation of cumene is usually effected by autoxidation with oxygen-containing gas such as the air, an oxygen-enriched air or the like. The oxidation reaction may be carried out without any additive or with an additive such as an alkali. The reaction temperature is usually 50 to 200°C, and the reaction pressure is usually between the atmospheric pressure and 5 MPa. In the oxidation using an additive, an alkali reagent used includes alkali metal compounds such as NaOH and KOH, alkaline earth metal compounds, alkali metal carbonates such as Na₂CO₃ and NaHCO₃, ammonia, (NH₄)₂CO₃, alkali metal ammonium carbonates and the like.

The epoxidation step is a step for obtaining propylene oxide and cumyl alcohol by reacting cumene hydroperoxide with propylene in an excess amount in a liquid phase in the presence of a solid catalyst for epoxidation. As the catalyst, from a viewpoint of obtaining the target product in high yield and under high selectivity, a solid catalyst containing a titanium-containing silicon oxide is preferable. The catalyst is preferably a so-called titanium-silica catalyst containing titanium chemically bound to silicon oxide. Examples thereof can include catalysts carrying a titanium compound on a silica carrier, catalysts in which a titanium compound is compounded with a silicon oxide by a co-precipitation or sol-gel method, titanium-containing zeolite compounds and the like.

Cumene hydroperoxide used as the raw material for the epoxidation step may be a dilute or thick purification or non-purification product.

The epoxidation is carried out by contacting propylene and cumene hydroperoxide with the catalyst. The reaction is conducted in a liquid phase using a solvent. The solvent should be a liquid under the reaction temperature and pressure, and substantially inert to the reactants and the product. The solvent may be composed of a substance existing in a solution of the hydroperoxide used. When, for example, cumene hydroperoxide is a mixture with cumene as the raw material, it is also possible to substitute cumene for a solvent, without adding a solvent in particular. Other useful solvents include aromatic monocyclic compounds (e.g. benzene, toluene, chlorobenzene, o-dichlorobenzene), alkanes (e.g. octane, decane, dodecane) and the like.

The epoxidation temperature is generally 0 to 200°C and preferably 25 to 200°C. The pressure may be any pressure enough to keep liquid state of the reaction mixture. Generally, the pressure is advantageously 100 to 10,000 kPa.

The solid catalyst can be advantageously used in the form of a slurry or a fixed-bed. The fixed-bed is preferred in the case of a large-scale industrial operation. In addition, the reaction can be carried out by a batch process, a semi-continuous process, a continuous process or the like. When a liquid containing the raw materials for reaction is passed through a fixed-bed, the catalyst is not contained at all or substantially in a liquid mixture discharged from a reaction zone.

The molar ratio of propylene to cumene hydroperoxide supplied to the epoxidation step, is preferably 2/1 to 50/1. When the ratio is less than 2/1, the efficiency may be deteriorated because of decrease of the reaction rate. On the other hand, when the ratio is more than 50/1, there is a tendency that energy required in the recovery step becomes large because of increase of a propylene amount to be recycled.

The hydrogenolysis step is a step for obtaining cumene by subjecting cumyl alcohol obtained in the epoxidation step to hydrogenolysis, and the produced cumene is recycled to the oxidation step as a raw material of the above-described oxidation step, and is as mentioned above. In addition, in the present invention, a step of separation removal of carbon monoxide and preferably further carbon dioxide is set up before supplying hydrogen as a raw material to the hydrogenolysis step.

### Example

The present invention is explained in detail by Examples, but is not limited thereto.

### Example 1

Into a fixed-bed flow reactor in which 100 g of a copper-silica catalyst was packed, a cumene solution containing 25% by weight of cumyl alcohol and hydrogen at rates of 2.7 g/minute and 16.66 normalized cm³/s (1000 Ncc/minute), respectively were fed under conditions of 1.4 MPaG and 217 °C. A reaction liquid obtained after 5 hours from the beginning of the feed, was analyzed, and the decomposition activity of cumyl alcohol was shown in Table 1.

### Example 2

A reaction was carried out in the same manner as in Example 1 except that hydrogen containing carbon monoxide of 1% by volume was fed into a fixed-bed reactor. The result was shown in Table 1.

### Comparative Example 1

A reaction was carried out in the same manner as in Example 1 except that hydrogen containing carbon monoxide of 10% by volume was fed into a fixed-bed reactor. The result was shown in Table 1.

**Table 1**

| | Example 1 | Example 2 | Comparative Example 1 |
|---|---|---|---|
| Carbon monoxide concontration(% by volume) | 0 | 1 | 10 |
| Cumyl alcohol decomposition activity(k) | 9.3 | 9.3 | 8.3 |

### Industrial Applicability

As described above, according to the present invention, aprocess forproducingcumene, which comprises subjecting cumyl alcohol to hydrogenolysis in the presence of a copper-based catalyst, and which has excellent characteristics that deterioration of an activity caused by reduction of a partial pressure of hydrogen and poisoning of the catalyst, can be prevented, whole volume of a reactor can be effectively utilized, and the amount purged of a gas can be suppressed to small when hydrogen is recycled, can be provided.

## Claims

1. A process for producing cumene, which comprises separating and removing carbon monoxide from a water gas to obtain hydrogen not containing carbon monoxide or containing 5% by volume or less of carbon monoxide and subjecting cumyl alcohol to hydrogenolysis using said hydrogen in the presence of a copper-based catalyst.

2. The process according to claim 1, wherein the total amount of carbon monoxide and carbon dioxide in the hydrogen used is 15% volume or less.

3. A process for producing propylene oxide, which comprises the following steps:
oxidation step: a step of obtaining cumene hydroperoxide by oxidizing cumene;
epoxidation step: a step of obtaining propylene oxide and cumyl alcohol by reacting cumene hydroperoxide obtained in the oxidation step with propylene in an excess amount in a liquid in the presence of a solid catalyst; and
hydrogenolysis step: a step of obtaining cumene by subjecting cumyl alcohol obtained in the epoxidation step to hydrogenolysis, and recycling the cumene to the oxidation step as a raw material for the oxidation step,
wherein the production of cumene is carried out by the process of claim 1 or claim 2.

## Patentansprüche

1. Verfahren zur Herstellung von Cumen, das die Abtrennung und Entfernung von Kohlenmonoxid von bzw. aus einem Wassergas zur Gewinnung von Wasserstoff, der kein Kohlenmonoxid enthält oder 5 Vol.-% oder weniger an Kohlenmonoxid enthält, und die Durchführung einer Hydrogenolyse von Cumylalkohol unter Verwendung des Wasserstoffs in Gegenwart eines Katalysators auf Kupferbasis umfasst.

2. Verfahren nach Anspruch 1, wobei die Gesamtmenge von Kohlenmonoxid und Kohlendioxid in dem verwendeten Wasserstoff 15 Vol.-% oder weniger beträgt.

3. Verfahren zur Herstellung von Propylenoxid, das die folgenden Stufen umfasst:
Oxidationsstufe: eine Stufe der Gewinnung von Cumenhydroperoxid durch Oxidation von Cumen;
Epoxidationsstufe: eine Stufe der Gewinnung von Propylenoxid und Cumylalkohol durch Umsetzung des in der Oxidationsstufe erhaltenen Cumenhydroperoxids mit Propylen in einer Menge im Überschuss in einer Flüssigkeit in Gegenwart eines festen Katalysators; und
Hydrogenolysestufe: eine Stufe der Gewinnung von Cumen durch Durchführen einer Hydrogenolyse des in der Epoxidationsstufe erhaltenen Cumylalkohols und der Rückführung des Cumens in die Oxidationsstufe als Ausgangsmaterial für die Oxidationsstufe,
wobei die Herstellung von Cumen durch das Verfahren nach Anspruch 1 oder Anspruch 2 durchgeführt wird.

## Revendications

1. Procédé pour produire du cumène, qui comprend la séparation et l'élimination du monoxyde de carbone à partir d'un gaz à l'eau pour obtenir de l'hydrogène ne contenant pas de monoxyde de carbone ou contenant 5 % en volume ou moins de monoxyde de carbone et la soumission d'alcool cumylique à une hydrogénolyse en utilisant ledit hydrogène en présence d'un catalyseur à base de cuivre.

2. Procédé selon la revendication 1, dans lequel la quantité totale de monoxyde de carbone et de dioxyde de carbone dans l'hydrogène utilisé est de 15 % en volume ou moins.

3. Procédé pour produire de l'oxyde de propylène, qui comprend les étapes suivantes :
étape d'oxydation : une étape d'obtention d'hydroperoxyde de cumène en oxydant du cumène ;
étape d'époxydation : une étape d'obtention d'oxyde de propylène et d'alcool cumylique en faisant réagir l'hydroperoxyde de cumène obtenu dans l'étape d'oxydation avec du propylène en une quantité d'excès dans un liquide en présence d'un catalyseur solide ; et
étape d'hydrogénolyse : une étape d'obtention de cumène en soumettant l'alcool cumylique obtenu dans l'étape d'époxydation à une hydrogénolyse, et en recyclant le cumène à l'étape d'oxydation en tant que matière première pour l'étape d'oxydation,
dans lequel la production de cumène est réalisée par le procédé selon la revendication 1 ou la revendication 2.
